# EUROPEAN PATENT SPECIFICATION

(11) **EP 4 236 814 B1**
(45) Date of publication and mention of the grant of the patent: **03.12.2025**
(21) Application number: 21810112.9
(22) Date of filing: 29.10.2021
(51) Int. Cl.: A61B 17/00

(54) **OCCLUSION DEVICE**
OKKLUSIONSVORRICHTUNG
DISPOSITIF D'OCCLUSION

(30) Priority: 02.11.2020 IT 202000026056
(43) Date of publication of application: 06.09.2023
(73) Proprietor: RECROSS CARDIO, INC., Kent County, DE 19901 (US)
(72) Inventor: ANTONUCCI, Marta, 20123 Milano (IT); GERHARDT, Thomas, Dover Kent County, DE 19901 (US)
(74) Representative: Crippa, Paolo Ernesto
(86) International application number: PCT/IB2021/060024
(87) International publication number: WO 2022/091019

(56) References cited:
- WO-A1-2017/136287
- US-A1- 2011 224 720
- US-A1- 2014 074 155
- US-A1- 2014 163 449
- US-A1- 2017 231 766

## Description

### . Field of the invention

**.** In the most general aspect thereof, the present invention relates to an occluder device. In particular, the present invention relates to an interseptal occluder device. Even more in particular, the present invention relates to an interseptal occluder device of the type which can be re-crossed.

**.** In particular, the present invention relates to a device for closing a defect of a septum or an opening obtained in a septum, not necessarily of the heart.

**.** Even more in particular, the present invention relates to a device for closing a defect present in a septum, for example a defect in an atrial septum, so that the same defect, even if occluded, can subsequently be used for the crossing of a medical, therapeutic and/or diagnostic device through said defect.

**.** Moreover, the device object of the present invention is intended to occlude defects (typically congenital defects, but not only) or interatrial septum holes/openings created following percutaneous interventions with trans-septal puncture techniques (for example for mitral valve repair or left atrial appendage occlusion).

**.** Moreover, the device object of the present invention is intended to reduce the left atrial pressure, in particular in subjects with cardiac diseases such as congestive heart failure (CHF) or myocardial infarction (MI).

### . Prior art

**.** A septum is for example a wall which divides a cavity into two smaller cavities or chambers or compartments. The term "septum" is intended to define both a heart wall which divides two atria as well as a wall which divides two ventricles.

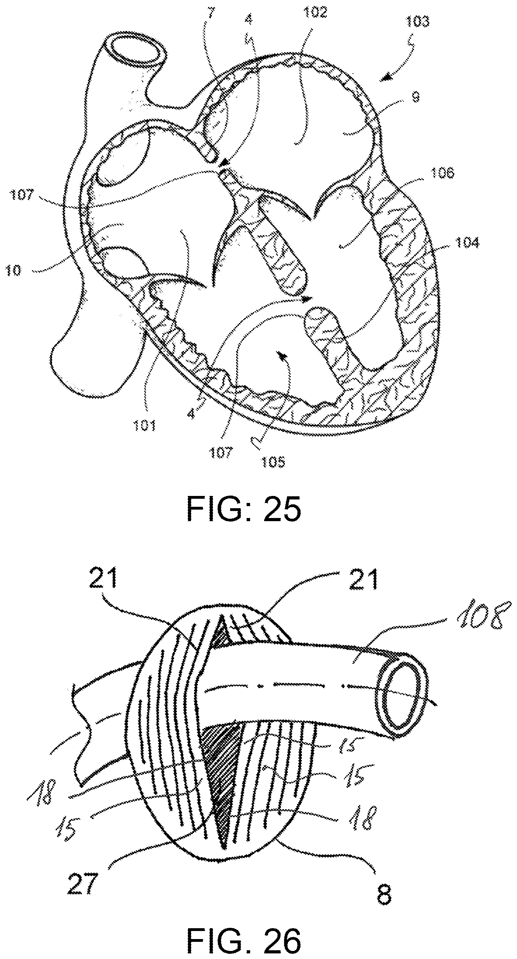

**.** Referring to figure 25, an atrial septum 100 is a tissue wall which separates the right atrium 101 from the left atrium 102 of the heart 103.

**.** A ventricular septum 104 is a tissue wall which separates the right 105 and left 106 ventricles of the heart 103.

**.** A defect 107 of the septum 100, 104 can include a perforation or hole in the septum. A defect 107 of the septum 100, 104 can occur congenitally or by perforating the septum with a medical device to access a location inside the heart.

**.** The femoral vein is an access point for many laboratory catheterization procedures, with a smaller percentage of procedures using the access to arteries.

**.** The atrial septum 100 is a percutaneous access point, for example, for the mapping and ablation therapy of cardiac arrhythmias such as atrial fibrillation, for closing the left atrial appendage, for the percutaneous repair of the mitral valve and for the percutaneous replacement of the mitral valve. In these and other procedures, the devices must cross the atrial septum 100 and, in doing so, can leave an orifice in the atrial septum which cannot close or heal on its own.

**.** Therefore, these defects are often closed using devices, such as clips or occluders. However, these devices do not allow re-crossing the septum if new procedures become necessary for the patient.

**.** Therefore, there is a need for improved occlusion devices for closing a defect or opening the septum, and for re-crossing it in (possible) subsequent procedures.

**.** Re-crossable occlusion devices are known in the prior art. These have an anchoring structure and a diaphragm or valve connected thereto.

**.** For example, documents WO2017136287A1, US2014074155 and US2014012368A1 show re-crossable occluder solutions.

**.** Document US20070073337 shows devices and methods for occluding the defects of internal tissues, such us septum defects, with clip-based devices. A device having a clip structure is shown, which includes a tubular body having at least a first and a second deflectable element coupled thereto. The first and second elements are coupled at the opposite ends of the tubular body and configured to switch from a non-deployed configuration to a deployed configuration. In the deployed configuration, each element extends outwards away from the tubular body in a position configured to rest on a tissue surface. The first and second elements of the clip are preferably configured to support a tissue wall therebetween and close any opening in the tissue wall. This solution does not allow re-crossing the device once implanted.

**.** Document US20140012368 shows devices and methods for improving the implantation, retractability, or repositioning of a device for positioning a valve in a septum. The embodiments of the devices include pivotable sections which provide the ability to maintain the engagement of the device with a release system during implantation, in which the release system approaches an opening of the septum. The embodiments of the devices include configurations which allow for better recovery in a delivery system if a malfunction or problem with the patient's anatomy or physiology is detected. This device is used to implant a flow control valve. These solutions are devices for treating heart failure. In particular, it is intended to obtain interatrial pressure outlets, shunts, and the like, which reduce the high pressure on one side of the heart, thus mitigating the resulting symptoms. Therefore, this solution aims to create an opening in the septum. This flow control element is a tissue valve such as a tricuspid valve, a bicuspid valve or a single-leaflet valve formed by pericardial tissue from cattle, pigs, sheep or other animals, and therefore it has cusp-like foils made of preferably natural tissue and cusp-shaped.

**.** Document US20160296684 shows endoluminal devices for treating heart failure, which include a central body adapted to allow the passage of interatrial blood, an anchor fixed to the central body adapted to keep the endoluminal device in place inside a defect present in the atrial septum of a patient's heart, and a control element rotationally engaged with the central body. The rotation of the control element in relation to the central body creates a bidirectional flow of interatrial blood which allows reducing the high pressure in the right and left parts of the heart. This solution has a valve which, depending on the rotating position of a diaphragm, the position of which has been previously fastened, allows a blood flow. This valve is obtained with a central body shutter, the rigidity of which allows the central body to keep a certain radial force outwards, against the septum walls, thus reducing the risk of displacement or migration of the endoluminal device.

**.** These solutions, although advantageous in some respects, are very complex to implement and above all do not allow completely occluding the septal defect and at the same time to freely choose the re-crossing point in different positions of the diaphragm. The possibility to accurately select such a re-crossing point is of considerable importance. In fact, the probability of success of trans-septal procedures, which address anatomical structures with very different positions with respect to the septum (such as pulmonary veins for atrial fibrillation, left auricle for the closure thereof, mitral annulus for repair or replacement of the mitral valve) is strongly influenced by the possibility for the operator to carefully choose the precise crossing point and the consequent spaces and relative maneuvering angles which such a point will allow. This is further reinforced by the fact that there is considerable variability in the size and location of such anatomical structures from patient to patient and from disease to disease.

**.** Therefore, the need for an occlusion device which is simple to manufacture, easy to apply, and flexible to use after implantation for crossing the defect or the now-occluded opening of the septum in case of subsequent surgery is still strongly felt.

**.** Furthermore, heart failure is the physiological state in which the cardiac output is insufficient to meet the needs of the body and lungs. Congestive heart failure occurs when cardiac output is relatively low and the body becomes congested by liquid. There are many possible underlying causes of CHF, including myocardial infarction, coronary heart disease, valvular disease, and myocarditis. Chronic heart failure is associated with neurohormonal activation and impaired autonomic control. Although these compensatory neurohormonal mechanisms provide valuable support for the heart under normal physiological circumstances, they also play a pivotal role in the development and subsequent progression of CHF. For example, one of the body's main compensatory mechanisms for reducing blood flow in CHF is to increase the amount of salt and water retained by the kidneys. Retaining salt and water, instead of excreting it in the urine, increases the volume of blood in the bloodstream and helps maintain blood pressure.

**.** However, the increased blood volume also stretches the heart muscle, enlarging the heart chambers, especially the ventricles. With a certain amount of stretching, the heart's contractions weaken and the heart failure worsens. Another compensatory mechanism is the vasoconstriction of the arterial system. This mechanism, like salt and water retention, increases blood pressure to help maintain adequate perfusion.

**.** In low ejection fraction (EF) heart failure, high pressures in the heart result from the body's attempt to maintain the high pressures necessary for adequate peripheral perfusion. However, the heart weakens due to the high pressures, aggravating the ailment. The pressure in the left atrium can exceed 25 mmHg, at which time the blood fluids flowing through the pulmonary circulatory system flow from the interstitial spaces and into the alveoli, causing pulmonary edema and pulmonary congestion.

**.** Various devices have been developed using stents or conduits to change blood pressure and the flow inside a given vessel or between the chambers of the heart.

**.** For example, patent US 6,120,534 by Ruiz relates to an endoluminal stent to regulate the flow of fluids through a vessel or organ of the body, for example to regulate blood flow through the pulmonary artery to treat congenital heart defects. The stent can include an expandable mesh having lobed or conical portions joined by a narrow region, which restricts flow through the stent. The mesh can comprise longitudinal struts connected by sinusoidal or coiled transverse connecting elements. However, Ruiz says nothing about treating CHF or reducing left atrial pressure.

**.** Publication US 6,468,303 by Amplatz et al. describes a collapsible medical device and associated method for deviating selected organs and vessels. Amplatz reveals that the device can be adapted to deflect a septal defect in a patient's heart, for example, creating a shunt in the atrial septum of a newborn with hypoplastic left heart syndrome (HLHS).

**.** Amplatz reveals that the increased mixing of pulmonary and systemic venous blood improves oxygen saturation.

**.** Amplatz reveals that, depending on the hemodynamics, the passage or shunt can subsequently be closed by an occlusion device.

**.** However, Amplatz says nothing about the treatment of CHF or the reduction of left atrial pressure, as well as the means to regulate the blood flow speed through the device.

**.** Publication US 2005/0165344 by Dobak describes an apparatus for treating heart failure which includes a conduit placed in a hole in the atrial septum of the heart, to allow the flow from the left atrium into the right atrium.

**.** Dobak reveals that the blood shunt will reduce left atrial pressures, thereby preventing pulmonary edema and progressive left ventricular dysfunction, and reducing LVEDP. Dobak reveals that the conduit can include a self-expanding tube with retention posts, such as metal arms which exert a slight force on the atrial septum on both sides and pinch or block the valve to the septum, and a one-way valve element, such as a tiltable disc, a bileaflet design or a flap valve formed from fixed animal pericardial tissue.

**.** However, Dobak says that a valve design may not be optimal due to the risk of blood stasis and thrombus formation on the valve, and that the valves can also damage blood components due to the effects of the turbulent flow.

**.** Dobak does not provide any specific guidance on how to avoid such problems.

**.** Publication US20110224720 by Cvdevices LLC also shows an occluder device designed specifically to create a seal, thus a complete occlusion, to the passage of blood (see for example paragraphs 96 and 97).

**.** This known solution, both for how it is made in the connection portion thereof to the wall or septum, and for the elongated shape thereof with the proximal end enlarged like a plug, is only adapted to form an occluder grippable for the positioning thereof. The construction thereof in one piece between the plug and the conical structure which is insertable in a septum opening makes it unadapted to withstand any stress which may arise in gripping it after the implantation and extraction thereof, showing the obvious risk that the sole gripping stress causes the device to pass over the opening with consequent loss beyond the septum with the occlusion (the size of the opening which requires the lateral notch to allow the divarication thereof during the insertion of an implant instrument, makes it clear how the friction generated between the device and the crossing shaft is of considerable intensity, placing the septum receiving it at risk of excessive stress where it is imagined as the opening of a second gripping after implantation. Thus this device cannot be crossed following the implantation thereof.

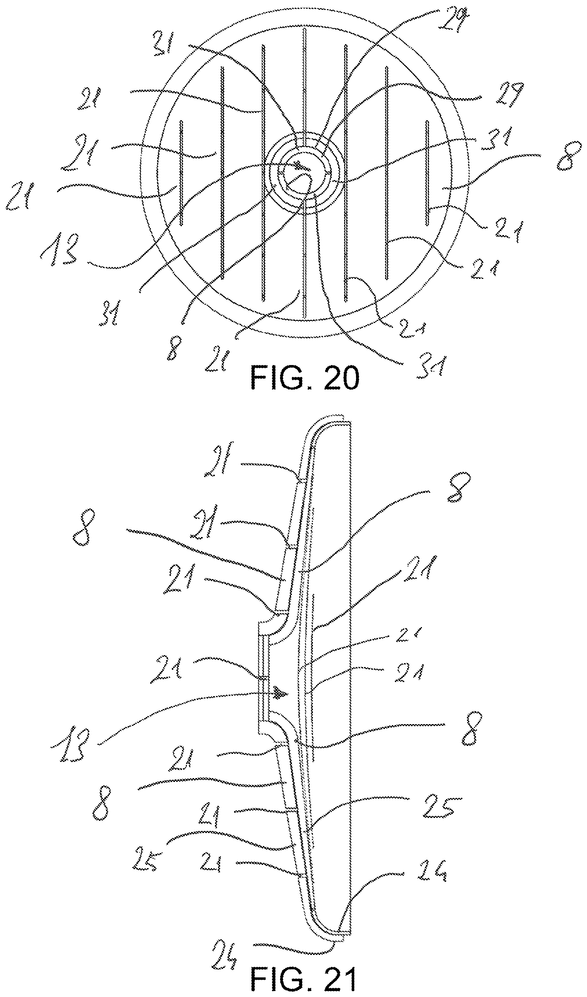

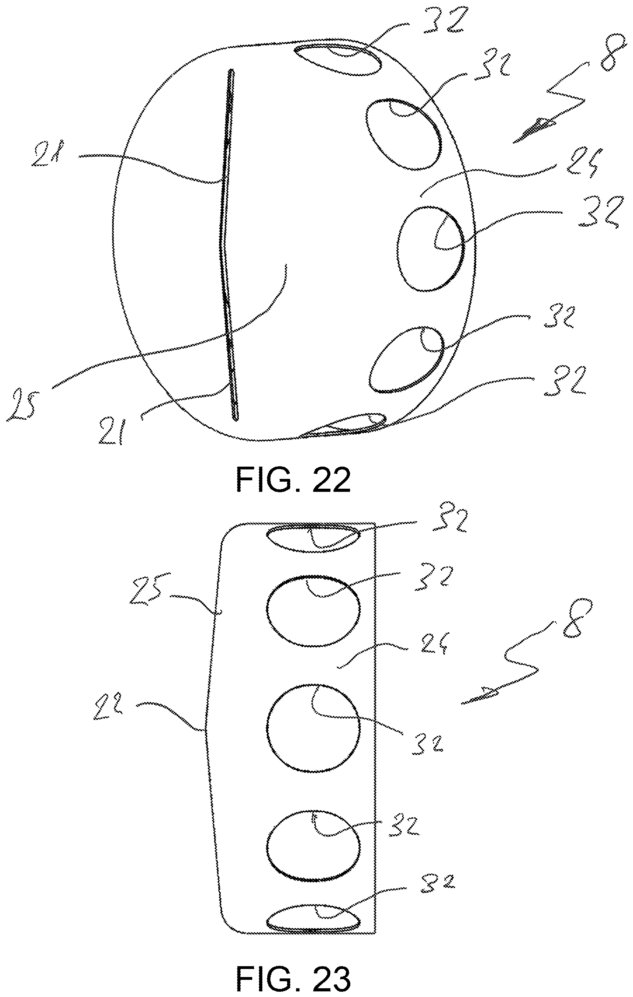

**.** Furthermore, this device, while showing a central gripping opening, is not designed to have a controlled passage adapted to remain open over time. In fact, in figures 21 and 22 a form is proposed having flaps perfectly closed and overlapping each other for a perfect seal against the passage of blood.

**.** Document US20170231766 by Mitraltech LTD shows a valve provided with flexible but not elastic petals, i.e., petals adapted to fold upon the arrival of a blood pressure wave, but not to extend, like an unsupported sheet of paper which folds under the force of wind which hits it but does not stretch elastically. This solution is not adapted to maintain a pervious opening even when in a closed configuration, since it is a valve and not a shunt.

**.** However, none of these documents allow creating a shunt in the septum and at the same time freely choosing the crossing point or re-crossing point in different positions of the diaphragm, facilitating the approach of the surgical instrument to the surgery area which can be located very differently from patient to patient and from disease to disease. Likewise, none of these documents allow the future possible re-crossing of the shunt with medical devices which have a dimension (diameter) which is considerably greater than the maximum diameter of the shunt itself.

### . Solution

**.** Therefore, it is the object of the present invention to provide a re-crossable interseptal occluder device which allows obtaining a shunt, having structural and functional features such as to meet the aforesaid needs and overcome the drawbacks mentioned above with reference to the devices of the prior art.

**.** These and other objects are achieved by a device according to claim **1.**

. Some advantageous embodiments are the subject of the dependent claims.

### . Drawings

**.** Further features and advantages of the invention will become apparent from the description provided below of preferred embodiments thereof, given by way of non-limiting examples, with reference to the accompanying drawings, in which:

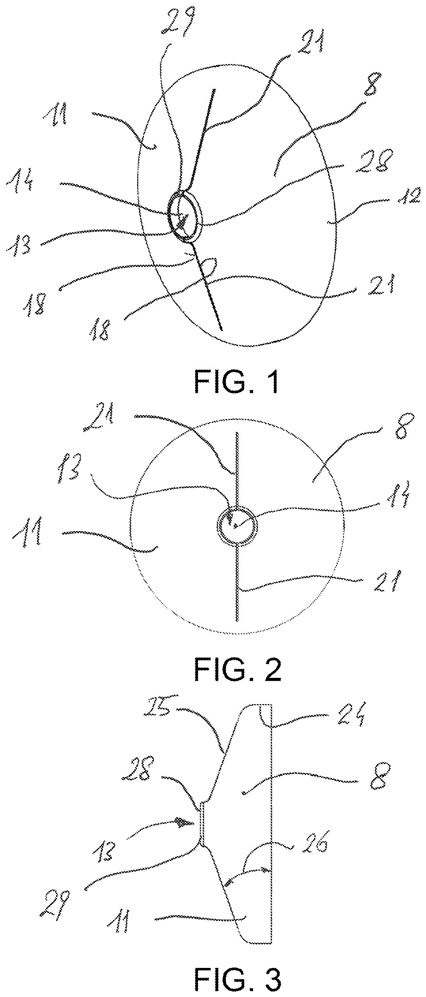

- figure 1 shows in an axonometric view a first embodiment of a membrane of an occlusion device according to the present invention;
- figure 2 depicts the front view of the device of figure 1;
- figure 3 shows the side view of the device of figure 1;

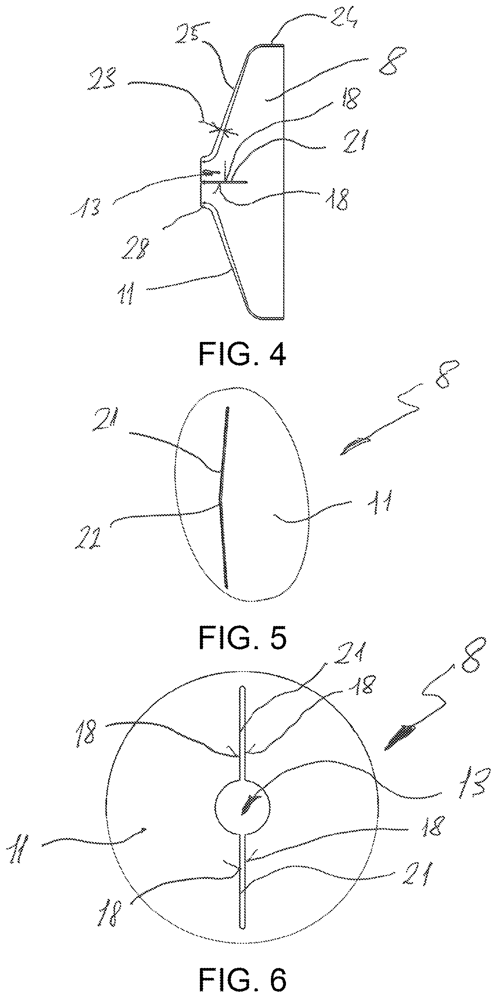

- figure 4 depicts the cross-section of the device of figure 1;
- figure 5 shows an axonometric view of a second embodiment of a membrane of a device of the invention;

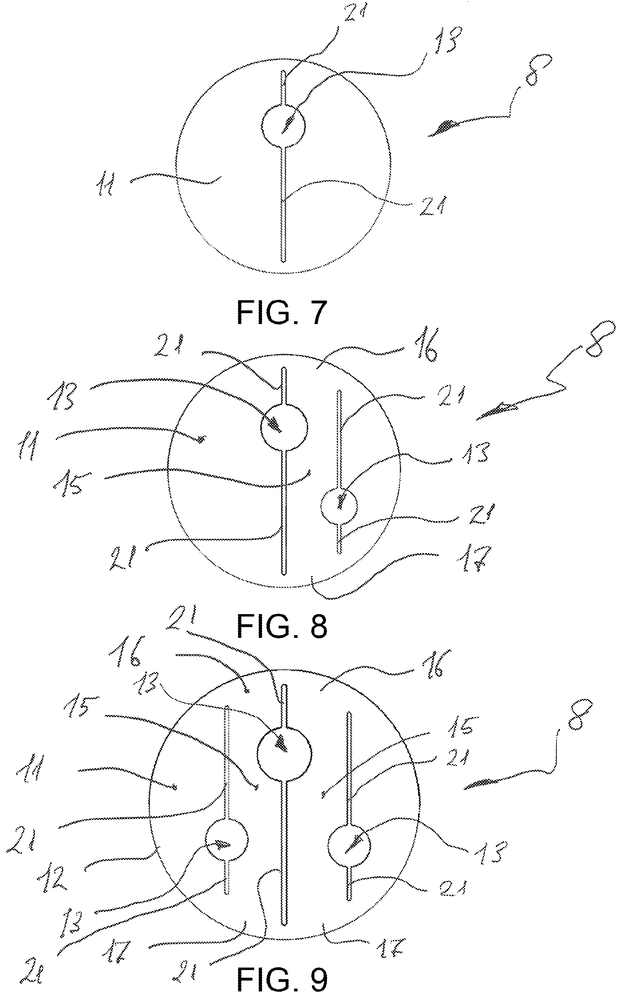

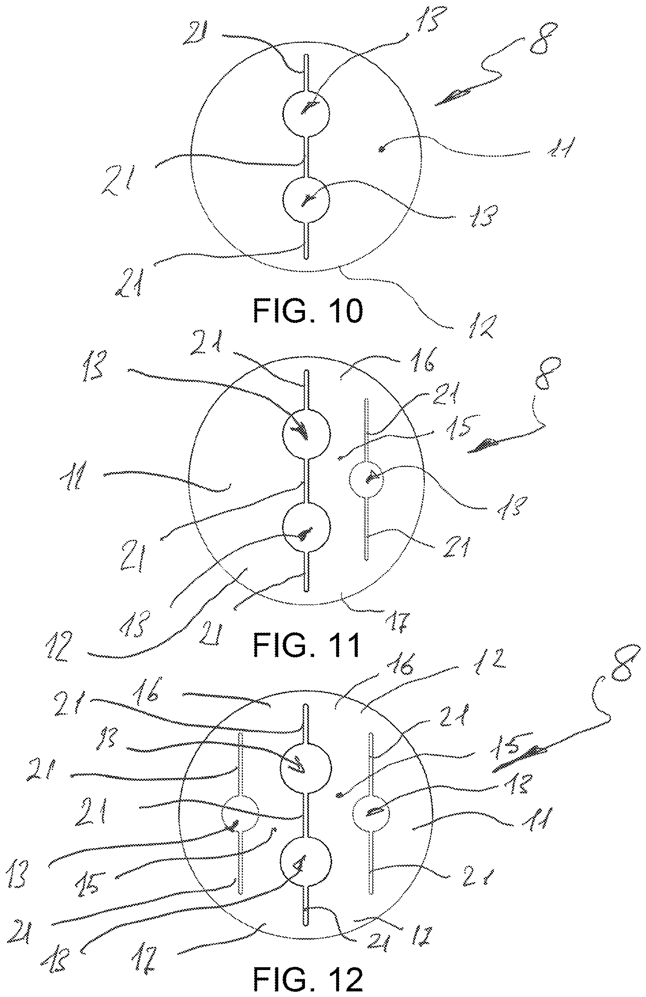

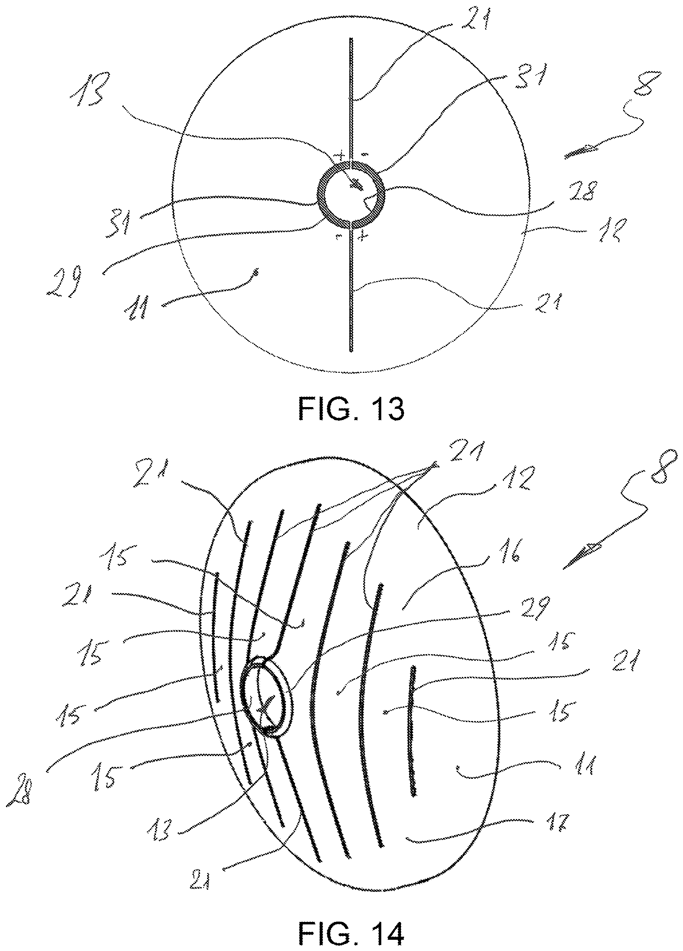

- figures 6 to 13 show a front view of eight further embodiments of a membrane of an occlusion device according to the present invention;
- figure 14 is an axonometric view of a still further embodiment of a membrane of an occlusion device according to the present invention;

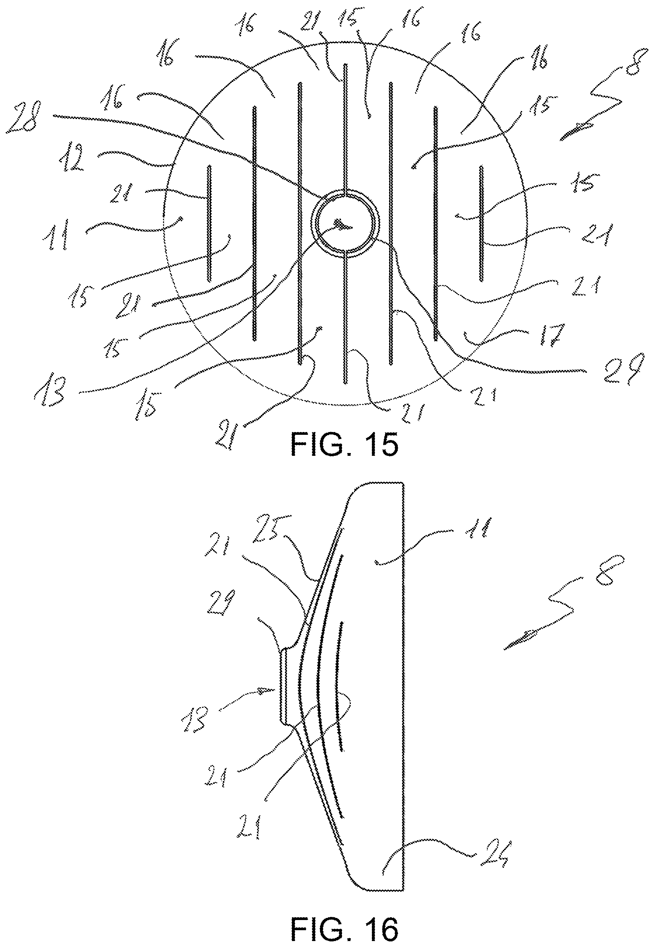

- figure 15 depicts the front view of the device of figure 14;
- figure 16 shows the side view of the device of figure 14;

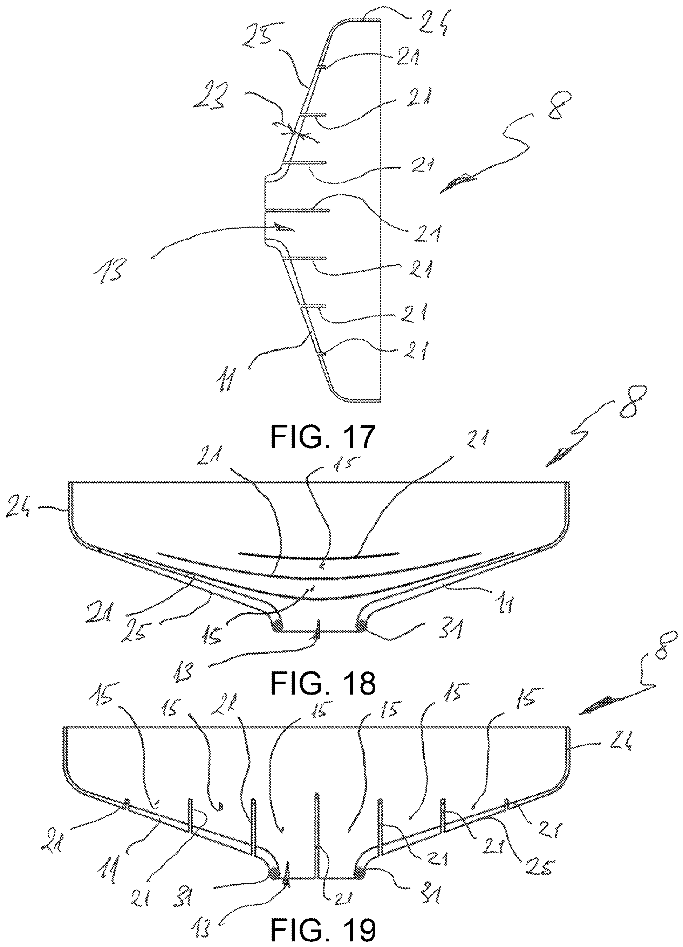

- figure 17 depicts the cross-section of the device of figure 14;
- figure 18 depicts the cross-section of a membrane of an occlusion device according to the present invention;
- figure 19 depicts the cross-section of the device of figure 18, but in a plane transverse to that of the section of figure 18;
- figure 20 shows a front view of a pair of membranes of an occlusion device according to the present invention;
- figure 21 shows the membranes of the device of figure 20 in cross-section;
- figure 22 shows an axonometric view of a membrane of an occlusion device according to a further embodiment of the present invention;
- figure 23 shows a side view of the membrane of the device of figure 22;

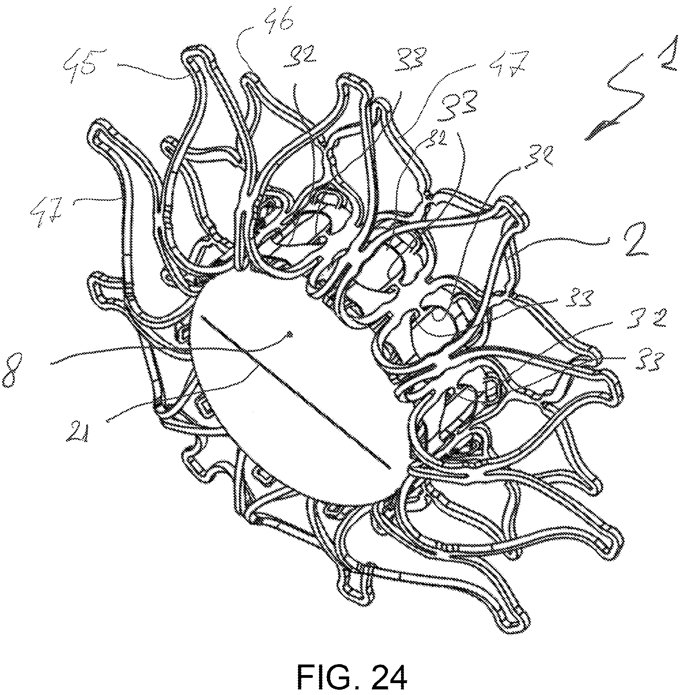

- figure 24 shows an axonometric view of an occlusion device according to an embodiment of the present invention;
- figure 25 depicts in section and diagrammatically a heart in which defects or openings are present in the atrial and ventricular septa;
- figure 26 diagrammatically shows in an axonometric view a step of the method of an external device crossing the membrane;

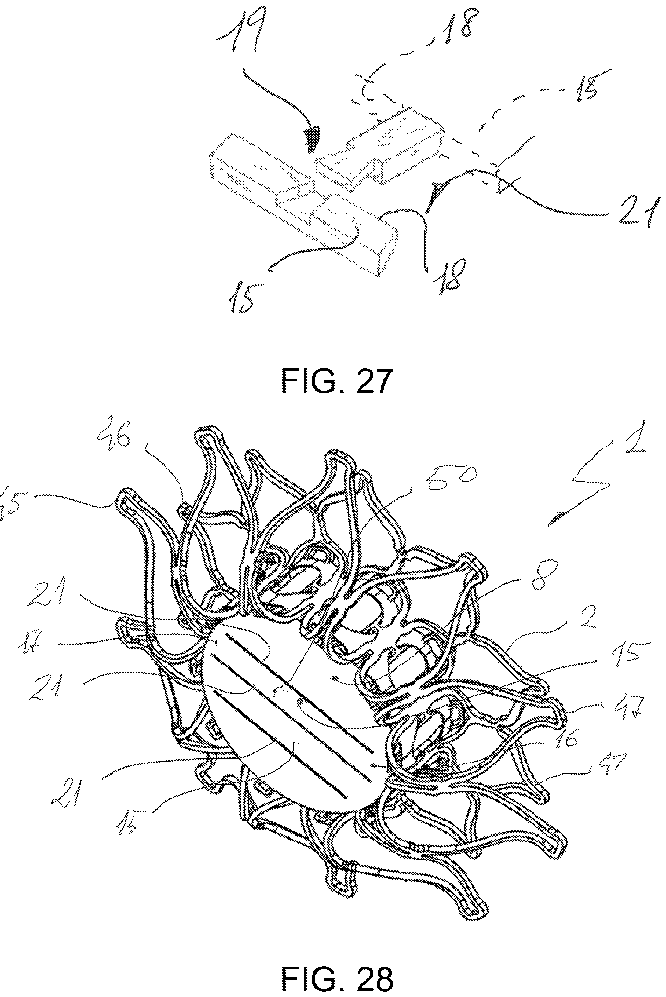

- figure 27 depicts an axonometric view of a detail of a geometric coupling between two edges of a membrane slit connected with a geometric coupling;
- figure 28 shows in an axonometric view an occlusion device according to a further embodiment of the present invention;

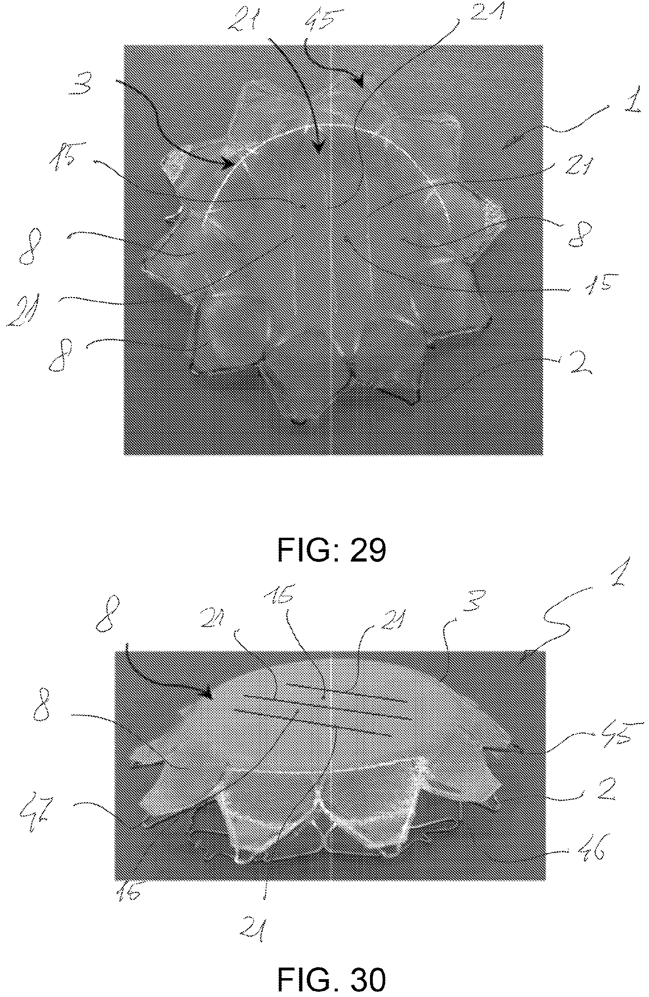

- figure 29 shows in a front axonometric view an occlusion device according to a still further embodiment of the present invention;
- figure 30 shows in a side axonometric view the occlusion device of figure 29.

### . Description of some preferred embodiments

**.** In accordance with a general embodiment, an occlusion device 1 comprises a supporting structure 2 adapted to be anchored to a septum 7 of a heart 103 when crossing a septum defect 107.

**.** Said supporting structure 2 comprises a supporting structure periphery 5 delimiting a structure central opening 6.

**.** Said occlusion device 1 further comprising a membrane 8.

**.** Said membrane 8 comprises a membrane body 11 having a membrane periphery 12.

**.** Said membrane 8 has a closed position in which the membrane 8 is in a configuration which provides maximum occlusion to the structure central opening 6.

**.** The membrane periphery 12 is fastened to said supporting structure periphery 5 to cover the structure central opening 6.

**.** Said membrane 8 comprising at least one slit 21.

**.** When actively engaged by an external device 108 the membrane 8 deforms the slit 21 which elastically extends, creating a passage lumen 27.

**.** Advantageously, said membrane 8 comprises at least one permanent passage lumen or shunt lumen 13 which remains open when the membrane 8 is in a closed position.

**.** Said at least one shunt lumen 13 is smaller with respect to said passage lumen 27.

**.** In accordance with an alternative embodiment, said at least one slit 21 extends from the membrane periphery 12 placed near the supporting structure 2.

**.** In accordance with an alternative embodiment, said at least one slit 21 extends with both ends thereof from the membrane periphery 12 placed near the supporting structure 2.

**.** In accordance with an alternative embodiment, said at least one slit 21 extends from a first membrane periphery portion 12 placed near the supporting structure 2 to a second membrane periphery portion 12 placed near the supporting structure 2.

**.** In accordance with an alternative embodiment, said at least one slit 21 extends from the membrane periphery 12 placed near the supporting structure 2 up to near the center of the membrane 14.

**.** In accordance with an alternative embodiment, said at least one slit 21 extends from the membrane periphery 12 near the supporting structure 2.

**.** In accordance with an alternative embodiment, said at least one slit 21 extends from the membrane periphery 12 near the supporting structure 2 at least to the center of the membrane 14.

**.** In accordance with an alternative embodiment, said at least one slit 21 is a notch.

**.** In accordance with an alternative embodiment, said at least one slit 21 are at least two slits 21.

**.** In accordance with an alternative embodiment, said at least one slit 21 are at least two slits 21 and said at least two slits 21 extend parallel to each other.

**.** In accordance with an alternative embodiment, said at least one slit 21 are at least two slits 21, said at least two slits 21 delimit a membrane portion forming a bridge 15 connecting two peripheral membrane portions 16, 17 separated from each other.

**.** In accordance with an alternative embodiment, said at least one slit 21 are cut edges or slotted edges 18 of the membrane 8.

**.** In accordance with an alternative embodiment, said at least one slit 21 comprises slit edges 18; said slit edges 18 are shaped and coupled, requiring separation force for the separation thereof, to keep the membrane 8 in a closed position during the normal operation thereof.

**.** In accordance with an alternative embodiment, said at least one slit 21 comprises slit edges 18; said slit edges 18 are geometrically coupled like a jigsaw piece or a dovetail joint 19.

**.** In accordance with an alternative embodiment, the shunt lumen 13 consists of said at least one slit 21; and said at least one slit 21 comprises slit edges 18 delimiting the shunt lumen 13, in which the slit edges 18 are separated creating an opening therebetween when the membrane 8 is in a closed position.

**.** In accordance with an alternative embodiment, the membrane 1, when actively engaged by an external device 108, elastically deforms to enlarge said at least one slit 21, obtaining said passage lumen 27.

**.** In accordance with an alternative embodiment, said shunt lumen 13 substantially maintains the dimension thereof during the normal operation of the heart 103 to maintain a defined blood flow when the occlusion device 1 is implanted through the septum defect 7 107.

**.** In accordance with an alternative embodiment, the cross-sectional area CSA of said shunt lumen 13 is an unobstructed lumen.

**.** In accordance with an alternative embodiment, the cross-sectional area CSA of said shunt lumen 13 is between 12 mm ^ 2 and 51 mm ^ 2.

**.** In accordance with an alternative embodiment, said membrane 8 defines an obstructed area of the structure central opening 6, said obstructed area is defined as the structure central opening 6 from which the cross-section area CSA of said shunt lumen 13 is subtracted.

**.** In accordance with an alternative embodiment, said membrane body 11 comprises a body structure 20 which with the normal operation of the heart 103 remains in a closed position avoiding substantially modifying the width of the shunt lumen 13.

**.** In accordance with an alternative embodiment, said membrane body 11 comprises a funnel shape.

**.** In accordance with an alternative embodiment, said membrane body 11 comprises a funnel shape and said at least one shunt lumen 13 is positioned at the vertex 22 of said funnel-shaped membrane body 11.

**.** In accordance with an alternative embodiment, said at least one slit 21 is a slit which crosses the entire membrane 8 from a portion 16 of the membrane periphery 12 to a different portion 17 of the membrane periphery 12.

**.** In accordance with an alternative embodiment, said membrane 8 has a circular shape and said at least one slit 21 is a slit which crosses the entire membrane 8 along a membrane diameter.

**.** In accordance with an alternative embodiment, the membrane body 11 has a membrane thickness 23.

**.** In accordance with an alternative embodiment, said membrane thickness 23 increases going towards the center of the membrane 8.

**.** In accordance with an alternative embodiment, said membrane 8 comprises a membrane periphery 12 having a cylindrical portion 24.

**.** Said cylindrical portion 24 comprising membrane anchoring seats 32.

**.** Said membrane anchoring seats 32 house clip portions 33 of said supporting structure 2 to connect the membrane 8 to said supporting structure 2.

**.** In accordance with an alternative embodiment, said membrane 8 comprises a truncated cone membrane portion 25 having a truncated cone shape.

**.** In accordance with an alternative embodiment, said membrane body 11 defines a membrane angle 26. Said membrane angle 26 is between 0°, i.e., the membrane is parallel to the septum 7, 100 or perpendicular to the blood flow, and 45° with respect to the septum 7, 100.

**.** In accordance with an alternative embodiment, said at least one shunt lumen 13 comprises shunt lumen edges 28. Said shunt lumen edges 28 are more rigid with respect to the remaining portions of the membrane body 11.

**.** In accordance with an alternative embodiment, said at least one shunt lumen 13 comprises shunt lumen edges 28. Said shunt lumen edges 28 comprise a greater thickness than the remaining portions of the membrane body 11.

**.** In accordance with an alternative embodiment, said at least one shunt lumen 13 comprises shunt lumen edges 28; said shunt lumen edges 28 are circular in shape.

**.** In accordance with an alternative embodiment, said membrane body 11 comprises at least one rib 29.

**.** In accordance with an alternative embodiment, said membrane body 11 comprises at least one rib 29; said at least one rib 29 is arranged next to said at least one shunt lumen 13.

**.** In accordance with an alternative embodiment, said membrane body 11 comprises at least one rib 29; said at least one rib 29 is arranged radially.

**.** In accordance with an alternative embodiment, said membrane body 11 comprises at least one rib 29; said at least one rib 29 is arranged circumferentially.

**.** In accordance with an alternative embodiment, said membrane body 11 comprises additional strips 30 arranged in a radial direction added radially to the membrane.

**.** In accordance with an alternative embodiment, said membrane 8 comprises magnets 31 embedded in the membrane.

**.** In accordance with an alternative embodiment, said membrane 8 comprises magnets 31, said magnets 31 are embedded in the membrane body 11 to keep the membrane 8 in a closed position during normal operation.

**.** In accordance with an alternative embodiment, said membrane 8 comprises magnets 31, said magnets are incorporated in the shunt lumen edges 28 of said shunt lumen 13 to keep the membrane 8 in a closed position during normal operation, leaving the shunt lumen 13 pervious.

**.** In accordance with an alternative embodiment, said at least one shunt lumen 13 are at least two shunt lumens 13.

**.** In accordance with an alternative embodiment, the at least one shunt lumen 13 are at least two shunt lumens 13 and said at least two shunt lumens 13 create at least two different openings through said membrane 8 of different size from each other.

**.** In accordance with an alternative embodiment, said at least one slit 21 opens into said at least one shunt lumen 13.

**.** In accordance with an alternative embodiment, said occluder device 1 comprises two membranes 8, in which said two membranes 8 are overlapped.

**.** In accordance with an alternative embodiment, said occluder device 1 comprises two membranes 8, in which said two membranes 8 are overlapped and supported at the same supporting structure 2.

**.** In accordance with an alternative embodiment, said occluder device 1 comprises two membranes 8, in which said two membranes 8 are overlapped and the at least one slit 21 of a first membrane 8 avoids completely overlapping the at least one slit 21 of the second membrane 8.

**.** In accordance with an alternative embodiment, said occluder device 1 comprises two membranes 8, in which said two membranes 8 are overlapped and the at least one slit 21 of a first membrane 8 extends along a first slit path, and the at least one slit 21 of a second membrane 8 extends along a second slit path.

**.** In accordance with an alternative embodiment, said first slit path crosses said second slit path.

**.** In accordance with an alternative embodiment, said occluder device 1 comprises two membranes 8, in which said two membranes 8 are overlapped and the at least one slit 21 of a first membrane 8 extends along a first slit path, and the at least one slit 8 of a second membrane extends along a second slit path.

**.** In accordance with an alternative embodiment, said first slit path crosses said second slit path in a region in which the first and second slit paths are orthogonal to each other when observing the membrane 8 from a transverse direction of said slit paths.

**.** In accordance with an alternative embodiment, said occlusion device 1 is a structure the purpose of which is to occlude a lumen 4, 107 to reduce the flow through said lumen 4, 107.

**.** In accordance with an alternative embodiment, said supporting structure 2 is a structure which provides the attachment to the anatomy of the heart 103, for example the tissue of a septum 7, 100, 104.

**.** In accordance with an alternative embodiment, said supporting structure periphery 5 is the peripheral edge of the structure 2 between the structure central opening 6 and the tissue of the septum 4, 100, 104.

**.** In accordance with an alternative embodiment, said structure central opening 6 is the central lumen of the supporting structure 2.

**.** In accordance with an alternative embodiment, said membrane 8 is an elastically flexible occlusive sheet.

**.** In accordance with an alternative embodiment, said membrane 8 is a flexible occlusive sheet with a thickness 23 which is much less than the height and width thereof.

**.** In accordance with an alternative embodiment, said closed position is the passive configuration of the membrane 8 where said membrane 8 occludes the maximum cross-sectional area of which it is capable.

**.** In accordance with an alternative embodiment, said membrane periphery 12 is the margin of the membrane 8 which separates the body of the membrane 11 from the supporting structure 2.

**.** In accordance with an alternative embodiment, said slit 21 is an unsupported edge of the membrane 8.

**.** In accordance with an alternative embodiment, said slit 21 is an unsupported edge of the membrane 8, or slit edge 18, said slit edge 18 is linear.

**.** In accordance with an alternative embodiment, said slit 21 is an unsupported edge of the membrane 8, or slit edge 18, said slit edge 18 is curved.

**.** In accordance with an alternative embodiment, said external device 108 is a medical device actively brought into apposition with the membrane 8, for example a catheter.

**.** In accordance with an alternative embodiment, said passage lumen 27, is a lumen inside the structure central opening 6 which is smaller than said structure central opening 6 but large enough to allow the passage of an external device 108.

**.** In accordance with an alternative embodiment, said shunt lumen 13 is a lumen inside the membrane 8 which remains pervious while the membrane 8 is in a closed, i.e., passive, position.

**.** In accordance with an alternative embodiment, said shunt lumen 13 is a lumen inside the membrane 8 which facilitates the transfer of blood between the heart chambers 9, 10, 101, 2102, 105, 106.

**.** A method for crossing an occluder device 1 comprises the following steps
- providing an occluder device 1 according to any one of claims 1 to 14;
- actively engaging an external device 108, for example a catheter, to the membrane 8;
- elastically deforming the at least one slit 21, elastically creating a passage lumen 27;
- crossing the occluding device 1 with said external device 108.

**.** In accordance with a variant of the method, a further step is included of:
- retracting the external device (108) allowing the membrane (8) to close in the original condition thereof.

**.** In accordance with an embodiment, said occluder device 1 comprises a membrane or diaphragm 8.

**.** Said membrane 8 is a flexible elastic sheet extending over both the structure central opening 6 and over the supporting structure 2 as well.

**.** In accordance with an embodiment, said membrane 8 extends beyond the supporting structure periphery 5, partially or totally covering at least one side of the supporting structure 2.

**.** Said supporting structure 2 comprises a central supporting structure portion 3 delimiting a lumen (4).

**.** Said supporting structure 2 comprises a first anchoring portion 45 and an opposite second anchoring portion (46).

**.** Said supporting structure 2 is configured to expand and contract between a compressed tubular configuration for the insertion through the patient's vasculature and an expanded configuration in which the first and the second anchoring portions 45, 46 extend radially outward from said central supporting structure portion 3 to compress a septum 100, 104 therebetween by arranging said supporting structure (2) astride said septum 100, 104 through a defect or hole or opening present in the septum 100, 104.

**.** In accordance with an embodiment, said first and second anchoring portions 45, 46 comprise a plurality of supporting structure arms 47 made of elastic material adapted to allow expanding and contracting between a compressed tubular configuration and an expanded or extended configuration for anchoring the arms to the opposite surfaces of the septum 7, 100, 104.

**.** In accordance with an embodiment, said supporting structure 2 is made of a less elastic material with respect to the material of said membrane 8. For example, said supporting structure is made of metallic material. For example, said supporting structure is made of metallic superelastic material.

**.** In accordance with an embodiment, said first and second anchoring portions 45, 46 comprise a plurality of supporting structure arms 47 made of elastic material adapted to allow expanding and contracting between a compressed tubular configuration and an expanded or extended configuration for anchoring the arms to the opposite surfaces of the septum 7, 100, 104.

**.** In accordance with an embodiment, said supporting structure 2 is in a separate piece or pieces with respect to said membrane 8. In accordance with an embodiment, said membrane 8 is connected to said supporting structure 2, for example said membrane 8 is fastened with sutures or is glued or is welded to said supporting structure 2.

**.** In accordance with an embodiment, said membrane 8 extends at least in part above one of said first and second anchoring portions 45, 46.

**.** In accordance with an embodiment, said at least one slit 21 extends at least partially above at least one of said first and second anchoring portions 45, 46.

**.** In accordance with an embodiment, said at least one slit 21 extends outside said structure central opening 6. This increases the maximum achievable length of the at least one slit 21, or in the case of at least two slits 21 of the bridge 15 present therebetween. By virtue of this embodiment, the stress which the material must undergo for a specific deformation is reduced. I.e., for a diameter of the external device 108, the longer the slit 21, the less stress the membrane 8 will undergo. Further, this embodiment reduces the force required to cross the slits 21 due to the lesser deformation of the membrane 8.

**.** In accordance with an embodiment, the membrane 8 is attached to a surface of the supporting structure 2 which is much wider with respect to the central supporting structure portion 3. This increases the strength of the bond between the membrane 8 and the supporting structure 3.

**.** The membrane 8 does not occlude the area delimited by the central supporting structure portion 3 when an external device 108 is pushed therethrough. This allows the external device 108 (e.g., a catheter) to be larger for a given central supporting structure portion 3, since the material of the membrane 8 is not limited during deformation by the central supporting structure portion 3.

**.** In accordance with an embodiment, if the membrane 8 extends beyond the central supporting structure portion 3 it reduces the angles, edges or shapes which could act as sites for thrombus formation or the creation of turbulence in the blood flow.

**.** In accordance with an embodiment, said membrane 8 comprises at least one bridge or occlusion bridge 15.

**.** Said at least one bridge 15 comprises an elongated bridge body 50 having opposite bridge connecting portions 16, 17 and opposite bridge edges coinciding with slit edges 18.

**.** Each of said slit edges 18 delimit at least one slit 21 present in said membrane 8.

**.** Both of said opposite bridge connecting portions 16, 17 are connected directly or indirectly with said supporting structure 2.

**.** In accordance with an embodiment, when said membrane 8 is in a relaxed condition, said membrane 8 is flat in shape and the at least one bridge 15 is lying flat on said supporting structure 2.

**.** In accordance with an embodiment, when said membrane 8 is in a relaxed condition, said membrane 8 is flat in shape and the at least one bridge 15 is lying tensioned on said supporting structure 2.

**.** In accordance with an embodiment, said at least one bridge 15 is made of deformable elastic material configured so as to extend when an external device 108 passes through the at least one slit 21.

**.** In accordance with an embodiment, said membrane 8 comprises at least two bridges or occlusion bridges 15.

**.** Said at least two bridges 15 each comprise an elongated bridge body 50 having opposite bridge connecting portions 16, 17 and opposite bridge edges coinciding with slit edges 18.

**.** Each of said slit edges 18 delimit with the slit edge 18 of the adjacent bridge at least one slit 21 present in said membrane 8.

**.** For each bridge 15, both of said opposite bridge connecting portions 16, 17 are directly or indirectly connected with said supporting structure 2.

**.** In accordance with an embodiment, when said membrane 8 is in a relaxed condition, said membrane 8 is flat in shape and the at least two bridges 15 are lying flat on said supporting structure 2.

**.** In accordance with an embodiment, when said membrane 8 is in a relaxed condition, said membrane 8 is flat in shape and the at least two bridges 15 are lying tensioned on said supporting structure 2.

**.** In accordance with an embodiment, said at least two bridges 15 are made of deformable elastic material configured so as to extend when an external device 108 passes through the at least one slit 21.

### LIST OF REFERENCES

- 1: occluder device
- 2: supporting structure
- 3: central supporting structure portion
- 4: septum lumen
- 5: supporting structure periphery
- 6: structure central opening
- 7: septum
- 8: membrane or diaphragm
- 9: first compartment
- 10: second compartment
- 11: membrane body
- 12: membrane periphery
- 13: shunt lumen or permanent passage lumen
- 14: membrane center
- 15: bridge
- 16: bridge connecting membrane periphery portion
- 17: bridge connecting membrane periphery portion
- 18: slit edges
- 19: dovetail joint
- 20: membrane body structure
- 21: membrane slit or cut
- 22: membrane vertex
- 23: membrane thickness
- 24: cylindrical membrane portion
- 25: conical membrane portion
- 26: membrane angle
- 27: passage lumen created with the slit by the elastic extension of the membrane
- 28: shunt lumen edges
- 29: membrane body rib
- 30: membrane strip
- 31: membrane magnets
- 32: membrane anchoring seats
- 33: supporting structure clip portions
- 45: supporting structure first anchoring portion
- 46: supporting structure second anchoring structure
- 47: supporting structure arms
- 50: elongated bridge body
- 100: atrial septum
- 101: right atrium
- 102: left atrium
- 103: heart
- 104: ventricular septum
- 105: right ventricle
- 106: left ventricle
- 107: defect or opening
- 108: external device

## Claims

1. An occluder device (1) comprising:
a supporting structure (2) adapted to anchor a septum (7) of a heart (103) of a patient when crossing a septum defect (107);
said supporting structure (2) comprising a central supporting structure portion (3) delimiting a lumen (4) and a supporting structure periphery (5) delimiting a structure central opening (6);
said occluder device (1) further comprising a membrane (8);
said membrane (8) comprises a membrane body (11) having a membrane periphery (12);
said membrane (8) has a closed position in which the membrane (8) is in a configuration which provides maximum occlusion to the structure central opening (6);
the membrane periphery (12) is fastened to said supporting structure periphery (5) to cover the structure central opening (6);
said membrane (8) comprising at least one slit (21);
when actively engaged by an external device (108) the membrane (8) deforms the at least one slit (21) which elastically extends, creating a passage lumen (27);
said membrane (8) comprises at least one permanent passage lumen or shunt lumen (13) which remains open when the membrane (8) is in a closed position; said at least one shunt lumen (13) is smaller with respect to said passage lumen (27);
wherein said supporting structure (2) comprises a first anchoring portion (45) and an opposite second anchoring portion (46); and
wherein said supporting structure (2) is configured to expand and contract between a compressed tubular configuration for insertion through the patient's vasculature and an expanded configuration in which the first and second anchoring portions (45, 46) extend radially outwards from said central supporting structure portion (3) to compress the septum therebetween by arranging said supporting structure (2) astride said septum through a defect or hole or opening present in the septum (100, 104).

2. The occluder device (1), according to claim 1, wherein said supporting structure (2) is made of a less elastic material with respect to a material of said membrane (8).

3. The occluder device (1), according to claim 1 or 2, wherein said first and second anchoring portions (45, 46) comprise a plurality of supporting structure arms (47) made of elastic material adapted to allow expanding and contracting between the compressed tubular configuration and the expanded configuration for anchoring the plurality of supporting structure arms to opposite surfaces of the septum (7, 100, 104).

4. The occluder device (1), according to any one of the preceding claims, wherein said supporting structure (2) is in a separate piece or pieces with respect to said membrane (8).

5. The occluder device (1), according to any one of the preceding claims, wherein
said at least one slit (21) extends from the membrane periphery (12) placed near the supporting structure (2); or wherein
said at least one slit (21) extends with both ends thereof from the membrane periphery (12) placed near the supporting structure (2); or wherein
said at least one slit (21) extends from a first membrane periphery portion (12) placed near the supporting structure (2) to a second membrane periphery portion (12) placed near the supporting structure (2).

6. The occluder device (1), according to any one of the preceding claims, wherein said at least one slit (21) extends from the membrane periphery (12) placed near the supporting structure (2) to near a center of the membrane (14).

7. The occluder device (1), according to any one of the preceding claims, wherein said at least one slit (21) is a notch.

8. The occluder device (1), according to any one of the preceding claims, wherein said at least one slit (21) are at least two slits (21).

9. The occluder device (1), according to claim 8, wherein said at least two slits (21) extend parallel to each other.

10. The occluder device (1), according to claim 8, wherein said at least two slits (21) delimit a membrane portion forming a bridge (15) connecting two membrane periphery portions (16, 17) separated from each other.

11. The occluder device (1), according to claim 10, wherein said bridge (15) comprises an elongated bridge body (50) having opposite bridge connecting portions (16, 17) and opposite bridge edges coinciding with slit edges (18).

12. The occluder device (1), according to claim 11, wherein each of said slit edges (18) delimits at least one slit (21) present in said membrane (8).

13. The occluder device (1), according to claim 11 or claim 12, wherein both said opposite bridge connecting portions (16, 17) are directly or indirectly connected to said supporting structure (2).

14. The occluder device (1), according to any one of claims 11 to 13, wherein when said membrane (8) is in a relaxed condition, said membrane (8) is flat in shape and the bridge (15) is lying flat on said supporting structure (2).

15. The occluder device (1), according to any one of claims 11 to 14, wherein said bridge (15) is made of deformable elastic material configured so as to extend when the external device (108) passes through the at least one slit (21).

## Patentansprüche

1. Okklusionsvorrichtung (1), umfassend:
eine Stützstruktur (2), die eingerichtet ist, ein Septum (7) eines Herzens (103) eines Patienten beim Durchqueren eines Septumdefekts (107) zu verankern;
wobei die Stützstruktur (2) einen zentralen Stützstrukturabschnitt (3) umfasst, der ein Lumen (4) begrenzt, und einen Stützstrukturrand (5), der eine Struktur-Zentralöffnung (6) begrenzt;
wobei die Okklusionsvorrichtung (1) ferner eine Membran (8) umfasst;
wobei die Membran (8) einen Membrankörper (11) mit einem Membranrand (12) umfasst;
wobei die Membran (8) eine Schließstellung aufweist, in welcher sich die Membran (8) in einer Konfiguration befindet, die einen maximalen Verschluss der Struktur-Zentralöffnung (6) bereitstellt;
wobei der Membranrand (12) an dem Stützstrukturrand (5) befestigt wird, um die Struktur-Zentralöffnung (6) zu bedecken;
wobei die Membran (8) mindestens einen Schlitz (21) umfasst;
wobei wenn sie aktiv mit einer externen Vorrichtung (108) aktiviert wird, verformt die Membran (8) den mindestens einen Schlitz (21), der sich elastisch erweitert und ein Durchgangslumen (27) schafft;
wobei die Membran (8) mindestens ein dauerhaftes Durchgangslumen oder Shunt-Lumen (13) umfasst, das offenbleibt, wenn sich die Membran (8) in einer Schließstellung befindet; wobei das mindestens eine Shunt-Lumen (13) kleiner im Vergleich zu dem Durchgangslumen (27) ist;
wobei die Stützstruktur (2) einen ersten Verankerungsabschnitt (45) und einen gegenüberliegenden zweiten Verankerungsabschnitt (46) umfasst; und
wobei die Stützstruktur (2) dazu konfiguriert ist, sich zwischen einer komprimierten rohrförmigen Konfiguration zum Einführen durch das Gefäßsystem des Patienten und einer expandierten Konfiguration, in der sich die ersten und zweiten Verankerungsabschnitte (45, 46) radial nach außen von dem zentralen Stützstrukturabschnitt (3) erstrecken, um das Septum dazwischen zu komprimieren, auszudehnen und zusammenzuziehen, indem die Stützstruktur (2) rittlings auf das Septum durch eine Defekt, ein Loch oder eine Öffnung, die in dem Septum (100, 104) vorhanden ist, angeordnet wird.

2. Okklusionsvorrichtung (1) nach Anspruch 1, wobei die Stützstruktur (2) aus einem weniger elastischen Material im Vergleich zu einem Material der Membran (8) hergestellt wird.

3. Okklusionsvorrichtung (1) nach Anspruch 1 oder 2, wobei die ersten und zweiten Verankerungsabschnitte (45, 46) eine Mehrzahl von Stützstrukturarmen (47) aus elastischem Material umfassen, die eingerichtet sind, ein Ausdehnen und Zusammenziehen zwischen der komprimierten rohrförmigen Konfiguration und der expandierten Konfiguration zu ermöglichen, um die Mehrzahl der Stützstrukturarme an gegenüberliegenden Oberflächen des Septums (7, 100, 104) zu verankern.

4. Okklusionsvorrichtung (1) nach einem der vorhergehenden Ansprüche, wobei die Stützstruktur (2) in einem separaten Teil oder in separaten Teilen im Vergleich zu der Membran (8) vorhanden ist.

5. Okklusionsvorrichtung (1) nach einem der vorhergehenden Ansprüche, wobei sich der mindestens eine Schlitz (21) von dem Membranrand (12), der in der Nähe der Stützstruktur (2) angeordnet ist, erstreckt; oder wobei
sich der mindestens eine Schlitz (21) mit beiden Enden von dem Membranrand (12), der in der Nähe der Stützstruktur (2) angeordnet ist, erstreckt;
oder wobei sich der mindestens eine Schlitz (21) von einem ersten Membranrandabschnitt (12), der in der Nähe der Stützstruktur (2) angeordnet ist, zu einem zweiten Membranrandabschnitt (12), der in der Nähe der Stützstruktur (2) angeordnet ist, erstreckt.

6. Okklusionsvorrichtung (1) nach einem der vorhergehenden Ansprüche, wobei der mindestens eine Schlitz (21) sich von dem Membranrand (12), der in der Nähe der Stützstruktur (2) angeordnet ist, bis in der Nähe eines Zentrums der Membran (14) erstreckt.

7. Okklusionsvorrichtung (1) nach einem der vorhergehenden Ansprüche, wobei der mindestens eine Schlitz (21) eine Kerbe ist.

8. Okklusionsvorrichtung (1) nach einem der vorhergehenden Ansprüche, wobei der mindestens eine Schlitz (21) mindestens zwei Schlitze (21) sind.

9. Okklusionsvorrichtung (1) nach Anspruch 8, wobei sich die mindestens zwei Schlitze (21) parallel zueinander erstrecken.

10. Okklusionsvorrichtung (1) nach Anspruch 8, wobei die mindestens zwei Schlitze (21) einen Membranabschnitt begrenzen, der eine Brücke (15) bildet, die zwei voneinander getrennte Membranrandabschnitte (16, 17) verbindet.

11. Okklusionsvorrichtung (1) nach Anspruch 10, wobei die Brücke (15) einen verlängerten Brückenkörper (50) aufweisend gegenüberliegende Brückenverbindungsabschnitten (16, 17) und gegenüberliegende Brückenrändern umfasst, die mit den Schlitzrändern (18) zusammenfallen.

12. Okklusionsvorrichtung (1) nach Anspruch 11, wobei jeder der Schlitzränder (18) mindestens einen in der Membran (8) vorhandenen Schlitz (21) begrenzt.

13. Okklusionsvorrichtung (1) nach Anspruch 11 oder Anspruch 12, wobei beide gegenüberliegenden Brückenverbindungsabschnitte (16, 17) direkt oder indirekt mit der Stützstruktur (2) verbunden sind.

14. Okklusionsvorrichtung (1) nach einem der Ansprüche 11 bis 13, wobei, wenn sich die Membran (8) in einem entspannten Zustand befindet, die Membran (8) flachförmig ist und die Brücke (15) flach auf der Stützstruktur (2) aufliegt.

15. Okklusionsvorrichtung (1) nach einem der Ansprüche 11 bis 14, wobei die Brücke (15) aus verformbarem elastischem Material hergestellt wird, das dazu konfiguriert ist, sich auszudehnen, wenn die externe Vorrichtung (108) durch den mindestens einen Schlitz (21) durchgeht.

## Revendications

1. Dispositif d'occlusion (1) comprenant :
une structure de support (2) conçue pour ancrer un septum (7) du cœur (103) d'un patient lors du franchissement d'un défaut septal (107) ;
ladite structure de support (2) comprenant une partie centrale de structure de support (3) délimitant une lumière (4) et une périphérie de structure de support (5) délimitant une ouverture centrale de structure (6) ;
ledit dispositif d'occlusion (1) comprenant en outre une membrane (8) ;
ladite membrane (8) comprend un corps de membrane (11) ayant une périphérie de membrane (12) ;
ladite membrane (8) présente une position fermée dans laquelle la membrane (8) est dans une configuration qui assure une occlusion maximale de l'ouverture centrale de la structure (6) ;
la périphérie de la membrane (12) est fixée à la périphérie de la structure de support (5) pour couvrir l'ouverture centrale de la structure (6) ;
ladite membrane (8) comprenant au moins une fente (21) ;
lorsqu'elle est activement engagée par un dispositif externe (108), la membrane (8) déforme la ou les fentes (21) qui s'étendent élastiquement, créant ainsi une lumière de passage (27) ;
ladite membrane (8) comprend au moins une lumière de passage permanente ou une lumière de dérivation (13) qui reste ouverte lorsque la membrane (8) est en position fermée ; ladite au moins une lumière de dérivation (13) est plus petite par rapport à ladite lumière de passage (27) ;
dans lequel ladite structure de support (2) comprend une première partie d'ancrage (45) et une seconde partie d'ancrage opposée (46) ; et
dans lequel ladite structure de support (2) est configurée pour se dilater et se contracter entre une configuration tubulaire comprimée pour l'insertion à travers le système vasculaire du patient et une configuration dilatée dans laquelle les première et deuxième parties d'ancrage (45, 46) s'étendent radialement vers l'extérieur à partir de ladite partie centrale de la structure de support (3) pour comprimer le septum entre elles en disposant ladite structure de support (2) à cheval sur ledit septum à travers un défaut, un trou ou une ouverture présent dans le septum (100, 104).

2. Le dispositif d'occlusion (1), selon la revendication 1, dans lequel ladite structure de support (2) est constituée d'un matériau moins élastique par rapport au matériau de ladite membrane (8).

3. Le dispositif d'occlusion (1), selon la revendication 1 ou 2, dans lequel lesdites première et deuxième parties d'ancrage (45, 46) comprennent une pluralité de bras de structure de support (47) en matériau élastique, adaptés pour permettre l'expansion et la contraction entre la configuration tubulaire comprimée et la configuration déployée, afin d'ancrer la pluralité de bras de structure de support sur des surfaces opposées du septum (7, 100, 104).

4. Le dispositif d'occlusion (1), selon l'une quelconque des revendications précédentes, dans lequel ladite structure de support (2) est constituée d'une ou de plusieurs pièces distinctes par rapport à ladite membrane (8).

5. Le dispositif d'occlusion (1), selon l'une quelconque des revendications précédentes, dans lequel ladite au moins une fente (21) s'étend à partir de la périphérie de la membrane (12) placée à proximité de la structure de support (2) ; ou dans lequel
ladite au moins une fente (21) s'étend avec ses deux extrémités depuis la périphérie de la membrane (12) placée à proximité de la structure de support (2) ; ou dans laquelle
ladite au moins une fente (21) s'étend d'une première partie périphérique de membrane (12) placée à proximité de la structure de support (2) à une seconde partie périphérique de membrane (12) placée à proximité de la structure de support (2).

6. Le dispositif d'occlusion (1), selon l'une quelconque des revendications précédentes, dans lequel ladite au moins une fente (21) s'étend de la périphérie de la membrane (12) placée près de la structure de support (2) jusqu'à près d'un centre de la membrane (14).

7. Le dispositif d'occlusion (1), selon l'une quelconque des revendications précédentes, dans lequel ladite au moins une fente (21) est une encoche.

8. Le dispositif d'occlusion (1), selon l'une quelconque des revendications précédentes, dans lequel ladite au moins une fente (21) est constituée d'au moins deux fentes (21).

9. Le dispositif d'occlusion (1), selon la revendication 8, dans lequel lesdites au moins deux fentes (21) s'étendent parallèlement l'une à l'autre.

10. Le dispositif d'occlusion (1), selon la revendication 8, dans lequel lesdites au moins deux fentes (21)
délimitent une portion de membrane formant un pont (15) reliant deux portions périphériques de membrane (16, 17) séparées l'une de l'autre.

11. Le dispositif d'occlusion (1), selon la revendication 10, dans lequel ledit pont (15) comprend
un corps de pont allongé (50) présentant des parties de connexion de pont opposées (16, 17) et des bords de pont opposés coïncidant avec les bords de fente (18).

12. Le dispositif d'occlusion (1), selon la revendication 11,
dans lequel chacun desdits bords de fente (18)
délimite au moins une fente (21) présente dans ladite membrane (8).

13. Le dispositif d'occlusion (1), selon la revendication 11 ou la revendication 12, dans lequel les deux dites
Les parties de connexion de pont opposées (16, 17) sont directement ou indirectement reliées à ladite structure de support (2).

14. Le dispositif d'occlusion (1), selon l'une quelconque des revendications 11 à 13, dans lequel, lorsque
ladite membrane (8) est dans un état détendu, ladite membrane (8) est de forme plate et le pont (15) repose à plat sur ladite structure de support (2).

15. Le dispositif d'occlusion (1), selon l'une quelconque des revendications 11 à 14, dans lequel ledit
Le pont (15) est constitué d'un matériau élastique déformable configuré de manière à s'étendre lorsque le dispositif externe (108) traverse au moins une fente (21).
